# EUROPEAN PATENT APPLICATION

(11) **EP 4 368 213 A1**
(43) Date of publication of application: **15.05.2024**
(21) Application number: 22837662.0
(22) Date of filing: 05.07.2022
(51) Int. Cl.: A61L 27/20, A61K 31/734, A61P 17/02

(54) **TISSUE FORMATION AGENT**

(30) Priority: 05.07.2021 JP 2021111683
(71) Applicant: NATIONAL UNIVERSITY CORPORATION SHIGA UNIVERSITY OF MEDICAL SCIENCE, Otsu-shi, Shiga 520-2192 (JP)
(72) Inventor: HAGIWARA, Akeo, Otsu-shi, Shiga 520-2192 (JP); TSUJIMOTO, Hiroyuki, Otsu-shi, Shiga 520-2192 (JP); HORII, Tsunehito, Otsu-shi, Shiga 520-2192 (JP); ISOGAI, Noritaka, Osaka-shi, Osaka 531-0071 (JP); HAGIWARA, Kohei, Otsu-shi, Shiga 520-0807 (JP); HAGIWARA, Yosaku, Osaka-shi, Osaka 550-0001 (JP)
(74) Representative: Duncombe, Jessica
(86) International application number: PCT/JP2022/026679
(87) International publication number: WO 2023/282247

(57) **Abstract**

An object of the present invention is to provide a tissue-forming agent. Provided is a tissue-forming agent comprising alginic acid or a salt thereof.

## Description

### Technical Field

The present invention relates to a tissue-forming agent, a tissue filler, and a tissue scaffolding agent.

### Background Art

Scaffolding agents and fillers are clinically used for the treatment of abnormalities in morphology, function, etc., including the healing of damage and wounds in the skin, mucous membrane, pleura, peritoneum, etc. caused by surgery, injury, disease, etc. As active ingredients of these, gelatin, collagen, hyaluronic acid, and the like are known (Non-patent Literature (NPL) 1 to 5).

Alginic acid, which is a polysaccharide found in algae, is a polymer composed of two types of uronic acids, i.e., mannuronic acid (M) and guluronic acid (G), as monomers, and there are polymers with various properties depending on the ratio of the two types of uronic acids (M/G ratio). Alginic acid is used in various fields such as food, pharmaceutical products, cosmetics, and thickening agents, and particularly in the field of pharmaceutical products, alginic acid is known to be usable as disintegrators for tablets, protective agents for the stomach wall, impression materials in the dental field, and the like.

### Citation List

### Non-patent Literature

NPL 1: Hajime Matsumine et al. "Full-thickness skin reconstruction with basic fibroblast growth factor-impregnated collagen-gelatin sponge" Regenerative Therapy 11, 81-87, 2019
NPL 2: Kuroda et al. "Clinical application of injectable growth factor for bone regeneration: a systematic review" Inflammation and Regeneration 39 (20), 2019
NPL 3: Chi H. Lee, Anuj Singla, Yugyung Lee "Biomedical applications of collagen" International Journal of Pharmaceutics 221, 1-22, 2001
NPL 4: Sayani Chattopadhyay and Ronald T. Raines "Collagen-Based Biomaterials for Wound Healing" Biopolymers. 101(8), 821-833, 2014
NPL 5: Alastair Carruthers, Jean Carruthers "Soft Tissue Augmentation E-Book: Procedures in Cosmetic Dermatology Series," Chapter 6, Berthold Rzany. "Belotero and Teosyal," pages 35-4

### Summary of Invention

### Technical Problem

The above-mentioned scaffolding agents and fillers, which contain gelatin, collagen, hyaluronic acid, or the like as an active ingredient, have weak cell infiltration and proliferation capacity in injection sites and thus do not exhibit satisfactory regenerative ability. They are also recognized as foreign substances at injection sites, causing inflammatory reactions.

### Solution to Problem

The present inventors conducted extensive research to solve the above problems and found that alginic acid or its sodium salt is useful as a tissue-forming agent. The inventors also found that alginic acid or its salt crosslinked with calcium is also useful as a tissue-forming agent. Furthermore, the inventors found that alginic acid or its sodium salt is useful as a tissue filler and a tissue scaffold agent. The present invention has been accomplished based on these findings and broadly includes the following subject matter.

### Item 1

A tissue-forming agent comprising alginic acid or a salt thereof.

### Item 2

The forming agent according to Item 1, wherein the tissue is an epithelial tissue, a tissue lining an epithelial tissue layer, or a tissue located deeper than these tissues.

### Item 3

The forming agent according to Item 1 or 2, wherein the amount of endotoxin contained is less than or equal to 1500 EU/mL or /mg.

### Item 4

The forming agent according to any of Items 1 to 3, wherein the alginic acid or a salt thereof has a viscosity of 0.1 to 1500 mPa/s.

### Item 5

The forming agent according to any of Items 1 to 4, wherein the alginic acid or a salt thereof has an M/G ratio of 0.001 to 0.999.

### Item 6

The forming agent according to any one of Items 1 to 5, wherein the alginic acid or a salt thereof is crosslinked with calcium or polylysine.

### Item 7

The forming agent according to any one of Items 1 to 6, wherein the alginic acid or a salt thereof is sulfated.

### Item 8

The forming agent according to any one of Items 1 to 7, further comprising a sulfated substance.

### Item 9

A tissue filler comprising alginic acid or a salt thereof.

### Item 10

A tissue scaffolding agent comprising alginic acid or a salt thereof.

### Item 11

A method comprising administering a composition containing alginic acid or a salt thereof to a patient in need of tissue formation.

### Item 12

A method comprising administering a composition containing alginic acid or a salt thereof to a patient in need of tissue regeneration.

### Item 13

A method comprising administering a composition containing alginic acid or a salt thereof to a patient in need of wound healing.

### Item 14

Use of alginic acid or a salt thereof in the production of a tissue-forming agent.

### Item 15

Use of alginic acid or a salt thereof in the production of a tissue filler.

### Item 16

Use of alginic acid or a salt thereof in the production of a tissue scaffolding agent.

### Item 17

A composition for tissue formation, comprising alginic acid or a salt thereof.

### Item 18

A composition for tissue regeneration, comprising alginic acid or a salt thereof.

### Item 19

A composition for wound healing, comprising alginic acid or a salt thereof.

### Advantageous Effects of Invention

The present invention provides a tissue-forming agent. The present invention also provides a tissue-forming agent that does not cause inflammation.

### Brief Description of Drawings

Fig. 1 shows the results of Example 1. Fig. 1(A) shows an HE stained image and an SOFG stained image 3 weeks after implantation of physiological saline. Fig. 1(B) shows an HE stained image and an SOFG stained image 3 weeks after implantation of AL2.
Fig. 2 shows the results of Example 2. Fig. 2(A) shows HE stained images and SOFG stained images 12 weeks after implantation of ALG20. Fig. 2(B) shows HE stained images and SOFG stained images 12 weeks after implantation of AL20.
Fig. 3 shows the results of Example 3. Fig. 3(A) shows HE stained images and SOFG stained images 12 weeks after implantation of ALG500. Fig. 3(B) shows HE stained images and SOFG stained images 12 weeks after implantation of AL500.
Fig. 4 shows the results of Example 4. Fig. 4(A) shows HE stained images and SOFG stained images 24 weeks after implantation of ALG20. Fig. 4(B) shows HE stained images and SOFG stained images 24 weeks after implantation of AL20.
Fig. 5 shows the results of Example 5. Fig. 5(A) shows HE stained images and SOFG stained images 24 weeks after implantation of ALG500. Fig. 5(B) shows HE stained images and SOFG stained images 24 weeks after implantation of AL500.
Fig. 6 shows the results of Example 6. Fig. 6(A) shows an HE stained image, an SOFG stained image, an MTC stained image, and a Col-I stained image 24 weeks after implantation of ALG20. Fig. 6(B) shows an HE stained image, an SOFG stained image, an MTC stained image, and a Col-I stained image 24 weeks after implantation of AL20.
Fig. 7 shows the results of Example 7. Fig. 7(A) shows an HE stained image, a Col-I stained image, a vWF stained image, and an alfa-SMA stained image 24 weeks after implantation of ALG20. Fig. 7(B) shows an HE stained image, a Col-I stained image, a vWF stained image, and an alfa-SMA stained image 24 weeks after implantation of AL20.
Fig. 8 shows the results of Example 8. Fig. 8(A) shows HE stained images, SOFG stained images, and MTC stained images 1 week after implantation of AL10. Fig. 8(B) shows HE stained images, SOFG stained images, and MTC stained images 1 week after implantation of AL100.
Fig. 9 shows the results of Example 9. Fig. 9(A) shows HE stained images, SOFG stained images, and MTC stained images 3 weeks after implantation of AL10. Fig. 9(B) shows HE stained images, SOFG stained images, and MTC stained images 3 weeks after implantation of AL100.
Fig. 10 shows the results of Example 10. Fig. 10(A) shows HE stained images, SOFG stained images, and MTC stained images 6 weeks after implantation of AL10. Fig. 10(B) shows HE stained images, SOFG stained images, and MTC stained images 6 weeks after implantation of AL100.
Fig. 11 shows the results of Example 11. Fig. 11(A) shows HE stained images, SOFG stained images, and MTC stained images 12 weeks after implantation of AL10. Fig. 11(B) shows HE stained images, SOFG stained images, and MTC stained images 12 weeks after implantation of AL100.
Fig. 12 shows the results of Example 12. Fig. 12(A) shows HE stained images, SOFG stained images, and MTC stained images 1 week after implantation of high-endotoxin AL20. Fig. 12(B) shows HE stained images, SOFG stained images, and MTC stained images 1 week after implantation of low-endotoxin AL20.
Fig. 13 shows the results of Example 13. Fig. 13(A) shows HE stained images, SOFG stained images, and MTC stained images 1 week after implantation of high-endotoxin AL500. Fig. 13(B) shows HE stained images, SOFG stained images, and MTC stained images 1 week after implantation of low-endotoxin AL500.
Fig. 14 shows the results of Example 14. Fig. 14(A) shows HE stained images, SOFG stained images, and MTC stained images 3 weeks after implantation of high-endotoxin AL20. Fig. 14(B) shows HE stained images, SOFG stained images, and MTC stained images 3 weeks after implantation of low-endotoxin AL20.
Fig. 15 shows the results of Example 15. Fig. 15(A) shows HE stained images, SOFG stained images, and MTC stained images 3 weeks after implantation of high-endotoxin AL500. Fig. 15(B) shows HE stained images, SOFG stained images, and MTC stained images 3 weeks after implantation of low-endotoxin AL500.
Fig. 16 shows the results of Example 16. Fig. 16(A) shows HE stained images, SOFG stained images, and MTC stained images 6 weeks after implantation of high-endotoxin AL20. Fig. 16(B) shows HE stained images, SOFG stained images, and MTC stained images 6 weeks after implantation of low-endotoxin AL20.
Fig. 17 shows the results of Example 17. Fig. 17(A) shows HE stained images, SOFG stained images, and MTC stained images 6 weeks after implantation of high-endotoxin AL500. Fig. 17(B) shows HE stained images, SOFG stained images, and MTC stained images 6 weeks after implantation of low-endotoxin AL500.
Fig. 18 shows the results of Example 18. Fig. 18(A) shows HE stained images, SOFG stained images, and MTC stained images 12 weeks after implantation of high-endotoxin AL20. Fig. 18(B) shows HE stained images, SOFG stained images, and MTC stained images 12 weeks after implantation of low-endotoxin AL20.
Fig. 19 shows the results of Example 19. Fig. 19(A) shows HE stained images, SOFG stained images, and MTC stained images 12 weeks after implantation of high-endotoxin AL500. Fig. 19(B) shows HE stained images, SOFG stained images, and MTC stained images 12 weeks after implantation of low-endotoxin AL500.
Fig. 20 shows the results of Comparative Example 1. Fig. 20(A) shows HE stained images, SOFG stained images, and MTC stained images 1 week after implantation of hyaluronic acid. Fig. 20(B) shows HE stained images, SOFG stained images, and MTC stained images 1 week after implantation of collagen.
Fig. 21 shows the results of Comparative Example 2. Fig. 21(A) shows HE stained images, SOFG stained images, and MTC stained images 3 weeks after implantation of hyaluronic acid. Fig. 21(B) shows HE stained images, SOFG stained images, and MTC stained images 3 weeks after implantation of collagen.
Fig. 22 shows the results of Comparative Example 3. Fig. 22(A) shows HE stained images, SOFG stained images, and MTC stained images 6 weeks after implantation of hyaluronic acid. Fig. 22(B) shows HE stained images, SOFG stained images, and MTC stained images 6 weeks after implantation of collagen.
Fig. 23 shows the results of Comparative Example 4. Fig. 23(A) shows HE stained images, SOFG stained images, and MTC stained images 12 weeks after implantation of hyaluronic acid. Fig. 23(B) shows HE stained images, SOFG stained images, and MTC stained images 12 weeks after implantation of collagen.
Fig. 24 shows the results of Example 20. Fig. 24(A) shows an HE stained image and an SOFG stained image 12 weeks after implantation of calcium-crosslinked AL10. Fig. 24(B) shows an HE stained image and an SOFG stained image 12 weeks after implantation of hyaluronic acid.
Fig. 25 shows the results of Example 21. Fig. 25(A) shows an HE stained image and an SOFG stained image 24 weeks after implantation of calcium-crosslinked AL10. Fig. 25(B) shows an HE stained image and an SOFG stained image 24 weeks after implantation of hyaluronic acid.
Fig. 26 shows the results of Example 22. Fig. 26(A) shows an HE stained image 1 week after implantation of sulfated sodium alginate. Fig. 26(B) shows an HE stained image 1 week after implantation of sodium alginate (AL100).
Fig. 27 shows the results of Example 23. Fig. 27(A) shows an HE stained image 2 weeks after implantation of a mixture of sodium alginate (AL10) and sulfated dextran. Fig. 27(B) shows an HE stained image 2 weeks after implantation of sodium alginate (AL10).
Fig. 28 shows the results of Example 24. Fig. 28(A) shows an HE stained image 1 week after implantation of calcium-crosslinked sodium alginate (AL10). Fig. 28(B) shows an HE stained image 1 week after implantation of sodium alginate (AL10).

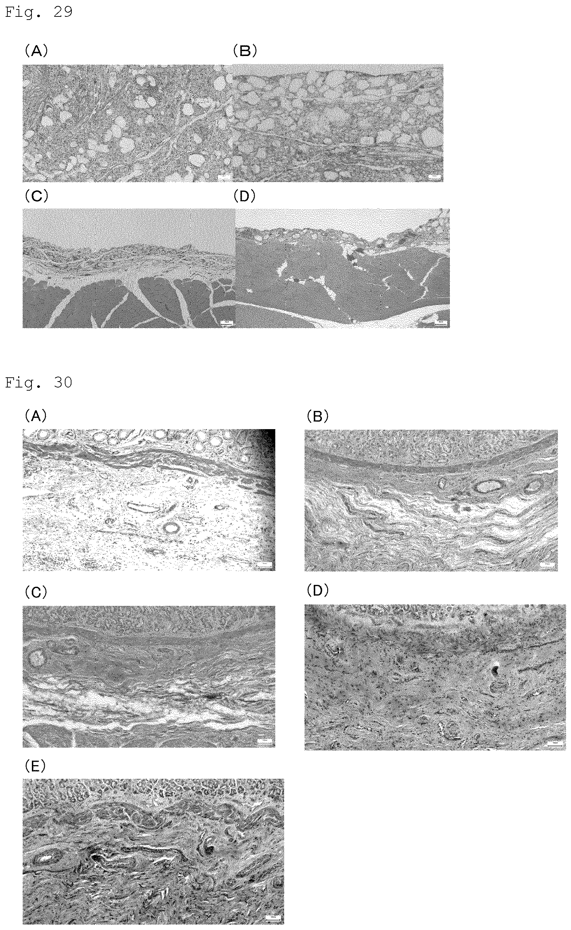

Fig. 29 shows the results of Example 25. Fig. 29(A) shows an HE stained image 4 weeks after implantation of a combination of calcium-crosslinked sodium alginate (AL10) and a PGA non-woven fabric into rat peritoneum. Fig. 29(B) shows an HE stained image 4 weeks after implantation of a combination of poly-L-lysine-crosslinked sodium alginate (AL10) and a PGA non-woven fabric into rat peritoneum. Fig. 29(C) shows an HE stained image 4 weeks after implantation of a PGA non-woven fabric alone into rat peritoneum. Fig. 29(D) shows an HE stained image 4 weeks after implantation of calcium-crosslinked sodium alginate (AL10) alone into rat peritoneum.
Fig. 30 shows the results of Example 26. Fig. 30(A) shows an HE stained image of an control experiment. Fig. 30(B) shows an HE stained image of a site in which relatively low-molecular-weight sodium alginate was injected. Fig. 30(C) shows an HE stained image of a site in which AL10 was injected. Fig. 30(D) shows an HE stained image of a site in which relatively low-molecular-weight alginic acid and a sodium polyglutamate solution were injected in the same site. Fig. 30(E) shows an HE stained image of a site in which AL10 and a sodium polyglutamate solution were injected in the same site.

### Description of Embodiments

Embodiments of the present invention are described in more detail below.

### Tissue-Forming Agent

The tissue-forming agent of the present invention comprises alginic acid or a salt thereof. The salt of alginic acid is not particularly limited as long as the effect of the present invention is exhibited. Specifically, the salt of alginic acid refers to one in which the hydrogen ions of the carboxyl groups of alginic acid are liberated, and cations are bonded. Examples of such cations include monovalent cations, such as sodium ions, potassium ions, and ammonium ions; and polyvalent cations, such as inorganic polyvalent ions (e.g., calcium ions, magnesium ions, iron ions, and ammonium ions) and organic polyvalent ions (e.g., polylysine). Of these salts, sodium salt is preferable in terms of water solubility.

The tissue in the tissue-forming agent of the present invention is not particularly limited. Specific examples include an epithelial tissue, a tissue lining an epithelial tissue layer, a tissue located deeper than these tissues, and the like.

The epithelial tissue is not particularly limited as long as the effect of the present invention is exhibited. Specific examples include mesothelial tissues such as the peritoneum, pleura, and pericardium, endothelial tissues such as blood vessels, lymphatic vessels, and meninges, as well as epithelial tissues in the narrow sense, such as the skin, urinary organs, genital organs, digestive organs, and respiratory organs. Of these, tissues such as the skin, digestive organs, uterus, bladder, mucosal tissues of trachea, kidneys, and liver are preferable.

The tissue lining an epithelial tissue layer is not particularly limited as long as the effect of the present invention is exhibited. Examples include the dermal tissue in the skin, the lamina muscularis mucosae or lamina propria in the mucosa of digestive organs, respiratory organs, urinary organs, or genital organs, and the like.

The tissue located deeper than the epithelial tissue and the tissue lining the epithelial tissue is not particularly limited as long as the effect of the present invention is exhibited. Examples include the subcutaneous tissue in the skin, the submucosal tissue in the mucosa, the peritoneal submesothelial layer, pleural submesothelial layer, etc. in mesothelial tissues, the subendothelial layer of the vascular intima in the endothelium, and the like.

The amount of endotoxin contained in the tissue-forming agent of the present invention is not particularly limited as long as the effect of the present invention is exhibited. Specifically, the amount of endotoxin contained in the tissue-forming agent of the present invention may be less than or equal to about 1500 EU/mL or /mg, preferably less than or equal to about 500 EU/mL or /mg, more preferably less than or equal to about 100 EU/mL or /mg, and most preferably less than or equal to about 50 EU/mL or /mg. The endotoxin measurement is performed by the method shown in the Bacterial Endotoxins Test of the Japanese Pharmacopoeia, 17th edition.

The viscosity of the alginic acid or salt thereof contained in the tissue-forming agent of the present invention is not particularly limited as long as the effect of the present invention is exhibited. Specifically, the viscosity of the alginic acid or salt thereof contained in the tissue-forming agent of the present invention may be about 0.1 to 1500 mPa/s, preferably about 2 to 500 mPa, more preferably about 50 to 100 mPa, and most preferably about 10 to 20 mPa.

The viscosity is measured under measurement conditions at 20°C in an aqueous solution having a concentration of 1% (mass).

The M/G ratio of the alginic acid or salt thereof contained in the tissue-forming agent of the present invention is not particularly limited as long as the effect of the present invention is exhibited. Specifically, the M/G ratio of the alginic acid or salt thereof contained in the tissue-forming agent of the present invention may be about 0.001 to 0.999.

The alginic acid or salt thereof contained in the tissue-forming agent of the present invention may also be crosslinked with, for example, a cation such as calcium, a polyvalent cation such as polylysine or acid-treated collagen, or a compounds in which a polysaccharide such as chitosan is in the form of a cation. The specific crosslinking method is not particularly limited as long as the effect of the present invention is exhibited. For example, the crosslinked alginic acid or salt thereof can be obtained by mixing alginic acid or a salt thereof with calcium, a compound in which a polysaccharide such as chitosan is in the form of a cation, or polylysine.

The calcium may be in any form as long as the effect of the present invention is exhibited. Specifically, the calcium may be, for example, in the form of salt. The calcium salt is not particularly limited as long as the effect of the present invention is exhibited. Examples include calcium salts of organic acids, calcium salts of inorganic acids, and the like. Specific examples include the calcium salt of gluconic acid.

The polylysine may be in any form as long as the effect of the present invention is exhibited. Specifically, the polylysine may be, for example, in the form of salt. The polylysine salt is not particularly limited as long as the effect of the present invention is exhibited. Examples include inorganic acid salts, such as hydrochloride, hydrobromide, and phosphate, organic acid salts, such as propionate, acetate, fumarate, malate, and citrate, and the like. The polylysine may be composed of D-lysine, L-lysine, or a mixture of D-lysine and L-lysine, and is preferably composed entirely of L-lysine.

The alginic acid or salt thereof contained in the tissue-forming agent of the present invention may be crosslinked with sulfuric acid. The specific sulfation method is not particularly limited as long as the effect of the present invention is exhibited. For example, sulfated alginic acid is prepared according to the method in "Whistler R.L. et al., Sulfates. Edited by Whistle R.L. et al., Methods in Carbohydrate Chemistry. Vol. II, 298-303. Academic Press (New York) (1963)."

More specifically, sodium alginate and a sulfur trioxide pyridine complex are stirred in dimethylformamide (at 40°C for 12 hours) to introduce sulfate groups into alginic acid, and sulfated alginic acid is precipitated and then neutralized and dissolved in a dilute aqueous sodium hydroxide solution, followed by dialysis for 72 hours and freeze-drying, thereby preparing sulfated alginic acid.

The tissue-forming agent of the present invention may consist essentially of the alginic acid or salt thereof, or may contain other components in addition to the alginic acid or salt thereof.

The other components are not particularly limited as long as the effect of the present invention is exhibited. Examples include pharmaceutically acceptable bases, carriers, additives (e.g., excipients, solvents, surfactants, preservatives, pH adjusters, and thickening agents), and the like. Such bases, carriers, additives, and the like are specifically described in, for example, Japanese Pharmaceutical Excipients Directory, and those described therein can be suitably used.

Moreover, a sulfated substance can be used together with the alginic acid or salt thereof. Such sulfated substances can be selected from those widely known as pharmaceutically acceptable sulfated substances. Examples include sulfated dextran, heparan sulfate (including heparin and heparinoid), chondroitin sulfate, keratan sulfate, carrageenan, funoran, dermatan sulfate, porphyrin, fucoidan, and the like. Of these, sulfated dextran or heparan sulfate is preferable.

The alginic acid and salt thereof exhibit a more excellent tissue-forming effect when used together with a biocompatible substance that exhibits strong adhesion to the surrounding area. The biocompatible substance that exhibits strong adhesion to the surrounding area is not particularly limited as long as the effect of the present invention is exhibited. Examples include pullulan, polyglutamic acid salts, and the like. Examples of other substances that can be used together to achieve a more excellent tissue-forming effect include thickeners (e.g., carrageenan, pectin, and gelatin), compounds in which polysaccharides, such as chitosan, are in the form of cations, polyvinylbenzyltrimethylammonium, and the like. Of these, pullulan, polyglutamic acid salts, and the like are preferable, and polyglutamic acid salts are most preferable.

The pharmaceutical form of the tissue-forming agent of the present invention is not particularly limited as long as the effect of the present invention is exhibited. For example, by using a usual method, alginic acid or a salt thereof and other components may be mixed, and the resulting mixture may be suitably placed in a wound or tissue or formulated into a preparation, such as an injection, according to the form of administration of the tissue-forming agent of the present invention.

The method for administering or placing the tissue-forming agent of the present invention is not particularly limited as long as the effect of the present invention is exhibited and it is a known administration method that is optimized for each dosage form. Specifically, the tissue-forming agent of the present invention can be administered or placed in, for example, the epithelium and adjacent tissues (epithelial tissues, mesothelial tissues, and endothelial tissues included in the epithelium in the broad sense, tissues lining these epithelial tissues, and tissues located deeper than these tissues) or in open wounds, closed wounds, adipose tissues, etc. at sites in contact with these tissues.

The site of administration or placement of the tissue-forming agent of the present invention is not particularly limited as long as the effect of the present invention is exhibited. For example, the tissue-forming agent of the present invention can be administered or placed in injury sites in the skin caused by surgery etc., wounded sites, or the epithelium and adjacent tissues or in other sites, such as open wounds, closed wounds, muscles, adipose tissues, joints, bone tissues, organs, and body cavities.

The dose of the tissue-forming agent of the present invention is determined according to the site in which tissue formation is desired, the degree of formation, and the degree of injury, and thus cannot be specified. As an example, the tissue-forming agent of the present invention may be administered in an amount of about 10% to 300% of the volume of the site per administration. The tissue-forming agent of the present invention is preferably administered in an amount of about 1000 ml or less per administration. This amount may be administered once a day or in several portions a day. In addition, the tissue-forming agent of the present invention may be administered several times per six months to per one month, several times per two weeks, or several times per week, or once every six, five, four, three, or two days.

### Tissue Filler

The tissue filler of the present invention comprises alginic acid or a salt thereof.

The alginic acid or salt thereof contained in the tissue filler of the present invention, the dose of the tissue filler, the administration method for the tissue filler, and the like may be the same as those of the tissue-forming agent of the present invention described above. Furthermore, the tissue targeted by the tissue filler may be the same as that of the tissue-forming agent of the present invention described above.

In particular, the alginic acid or salt thereof contained in the tissue filler of the present invention tends to be more highly crosslinked than the alginic acid or salt thereof contained in the tissue-forming agent of the present invention described above.

### Tissue Scaffolding Agent

The tissue scaffolding agent of the present invention comprises alginic acid or a salt thereof.

The alginic acid or salt thereof contained in the tissue scaffolding agent of the present invention, the dose of the tissue scaffolding agent, the administration method for the tissue scaffolding agent, and the like may be the same as those of the tissue-forming agent of the present invention described above. Furthermore, the tissue targeted by the tissue scaffolding agent may be the same as that of the tissue-forming agent of the present invention described above.

The tissue scaffolding agent of the present invention may comprise a conventionally known tissue reinforcement material (also referred to as an "organ defect reinforcement material") together with the alginic acid or salt thereof. Such tissue reinforcement materials can be selected from those widely known as pharmaceutically acceptable tissue reinforcement materials. Examples include non-woven fabrics containing polyglycolic acid, polylactic acid, or some components thereof, such as a non-woven fabric of polyglycolic acid (PGA non-woven fabric) and a non-woven fabrics of a copolymer of lactic acid and caprolactone (LA/CL non-woven fabric); non-woven fabrics containing a component derived from an organism, such as a polyvinyl component, a polyurethane component, a cellulose component, collagen, or gelatin; and the like. Of these tissue reinforcement materials, PGA non-woven fabrics, LA/CL non-woven fabrics, and the like are preferable.

In the present specification, the expression "containing" or "comprising" a component not only means that the component is contained and that other components may be further contained, but also includes the concept of "consisting of," which means that only the component is contained, and the concept of "consisting essentially of," which means that the component is essentially contained.

The contents of the references and web pages mentioned herein are incorporated herein by reference.

Various characteristics, such as properties, structures, and functions, described in the above embodiments of the present invention may be combined as appropriate to specify the embodiments included in the present invention. That is, the present invention includes all of the subject matter of embodiments of the combinable properties described herein.

### Examples

The present invention is described in more detail below with reference to the Examples described below; however, the present invention is not limited to these Examples.

The reagents used in the following Examples are described below.

- Physiological saline: Otsuka Normal Saline 20-mL plastic ampoule, product number (YJ code): 3311401A2026
- Various kinds of sodium alginate (produced by Kimica Corporation)
- Low-endotoxin sodium alginate AL2 (weight average molecular weight: 7,700 kDa; viscosity 1% aqueous solution)
- Low-endotoxin sodium alginate AL10 (weight average molecular weight: 57 kDa; viscosity 1% aqueous solution: 11 mPa/s)
- Low-endotoxin sodium alginate AL20 (weight average molecular weight: 91 kDa; viscosity 1% aqueous solution: 20-25 mPa/s)
- Low-endotoxin sodium alginate AL100 (weight average molecular weight: 170-180 kDa; viscosity 1% aqueous solution: 100-120 mPa/s)
- Low-endotoxin sodium alginate AL500 (weight average molecular weight: 280-300 kDa; viscosity 1% aqueous solution: 400-600 mPa/s)
- Low-endotoxin sodium alginate ALG2 (weight average molecular weight: 7,7 kDa; viscosity 1% aqueous solution)
- Low-endotoxin sodium alginate ALG20 (weight average molecular weight: 91 kDa; viscosity 1% aqueous solution: 20-25 mPa/s)
- Low-endotoxin sodium alginate ALG500 (weight average molecular weight: 280-300 kDa; viscosity 1% aqueous solution: 400-600 mPa/s)

The amount of endotoxin contained in each kind of low-endotoxin sodium alginate described above is ≤50EU.

- High-endotoxin sodium alginate AL20 (weight average molecular weight: 91 kDa; viscosity 1% aqueous solution: 30 mPa/s)
- High-endotoxin sodium alginate AL500 (weight average molecular weight: 280-300 kDa; viscosity 1% aqueous solution: 400 mPa/s)

The amount of endotoxin contained in each kind of high-endotoxin sodium alginate described above is ≥10000EU.

- Koken Atelocollagen Implant (Koken Co., Ltd.; product number: #1332)
- Hyaluronic acid (for joint injection), product number (product name code): 3999408G1417 Sodium Hyaluronate for Joint Injection, 25-mg Syringe (Nichi-Iko Pharmaceutical Co., Ltd.), 25 mg/2.5 ml
- TEOSYAL RHA 1 (hyaluronic acid), Teoxane, product number (product ID): 1208crosslinker, rate: 1.9%

### Example 1

Six rats (Wistar/ST female) were anesthetized by intraperitoneal administration of pentobarbital and inhalation of isoflurane, and each of the solutions (physiological saline and a 2% aqueous solution of low-endotoxin sodium alginate AL2) was individually injected (implanted) in an amount of 1 ml into the tissue between the subcutaneous fascia at a subcutaneous depth of about 1 cm at each of eight sites from the upper back to the lower back, using 1-ml syringe and a 23G needle. The injection of each solution was performed after randomization. Each needle entry site was sutured with one stitch of 5-0 proline thread.

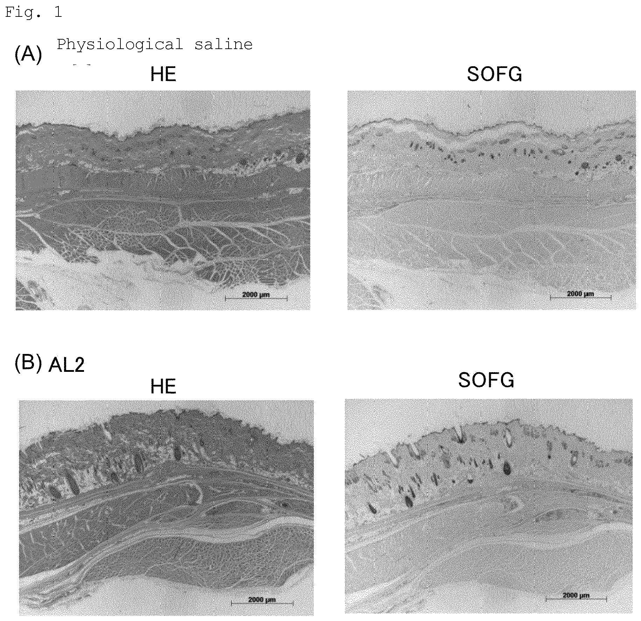

After 3 weeks, each rat was sacrificed by pentobarbital overdose, and the back implantation sites were cut out and immersion-fixed in a 10% formaldehyde solution. The fixed tissues were subjected to hematoxylin-eosin staining (HE staining) and safranin-O staining (SOFG staining) according to usual methods. Fig. 1 shows the results.

The results in Fig. 1 show that AL2 disappeared and no inflammatory changes were observed.

### Example 2

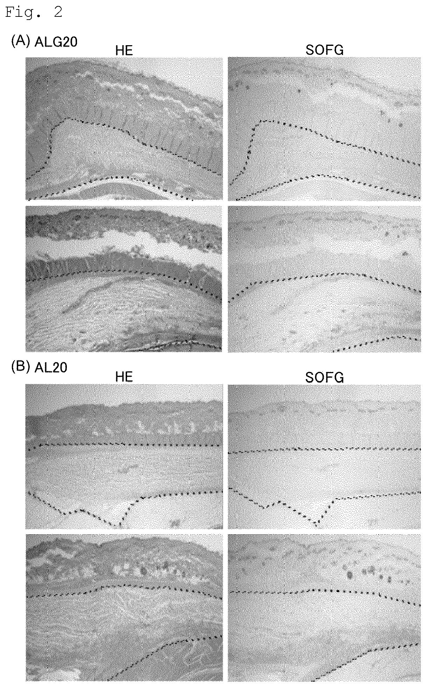

Each of 2% aqueous solutions of low-endotoxin sodium alginate ALG20 and low-endotoxin sodium alginate AL20 was individually implanted in an amount of 1 ml into six rats in a manner similar to that in Example 1. After 12 weeks, HE staining and SOFG staining were performed in the same manner as in Example 1. Fig. 2 shows the results.

The results in Fig. 2 show that in the implantation of ALG20 and the implantation of AL20, there was a small amount of retention, and it was believed that there was a change in orientation in both. Inflammatory changes were reduced, but they persisted in some cases. No particular differences were found between ALG20 and AL20.

### Example 3

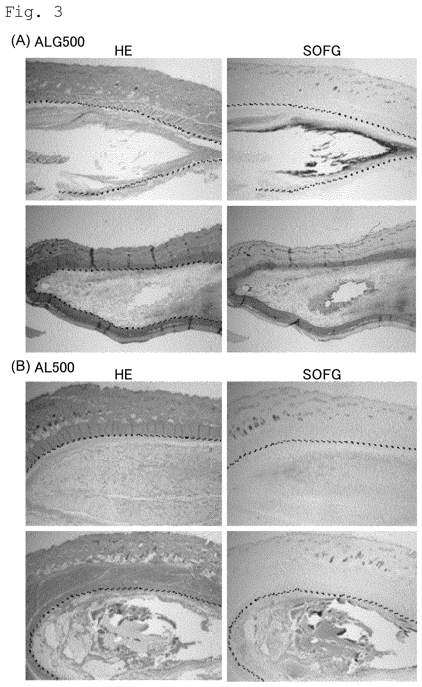

Each of 2% aqueous solutions of low-endotoxin sodium alginate ALG500 and low-endotoxin sodium alginate AL500 was individually implanted in an amount of 1 ml into six rats in a manner similar to that in Example 1. After 12 weeks, HE staining and SOFG staining were performed in the same manner as in Example 1. Fig. 3 shows the results.

The results in Fig. 3 show that in the implantation of ALG500 and the implantation of AL500, there was a large amount of retention. Inflammatory changes were reduced, but they strongly persisted in some cases. No particular differences were found between ALG500 and AL500.

### Example 4

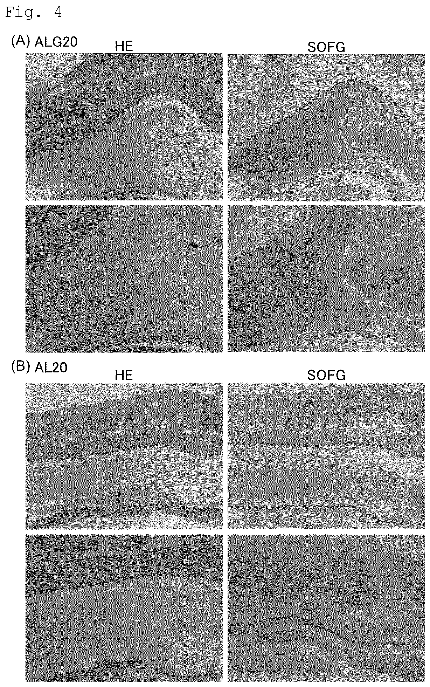

Each of 2% aqueous solutions of low-endotoxin sodium alginate ALG20 and low-endotoxin sodium alginate AL20 was individually implanted in an amount of 1 ml into six rats in a manner similar to that in Example 1. After 24 weeks, HE staining and SOFG staining were performed in the same manner as in Example 1. Fig. 4 shows the results.

The results in Fig. 4 show that in the implantation of ALG20 and the implantation of AL20, there was a small amount of retention, and it was believed that there was a significant change in orientation in both. Inflammatory changes were reduced, but they persisted in some cases. No particular differences were found between ALG20 and AL20.

### Example 5

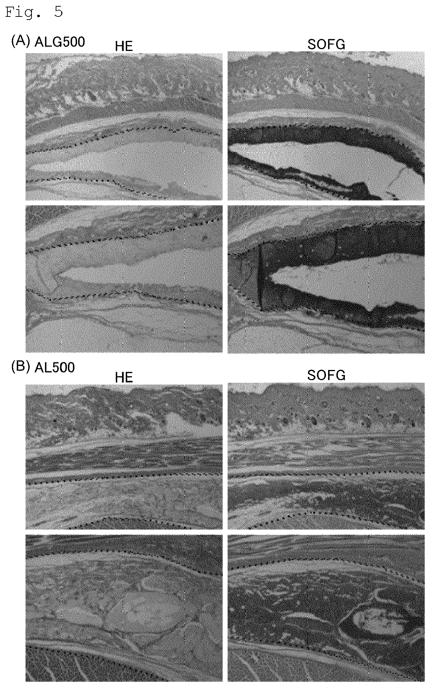

Each of 2% aqueous solutions of low-endotoxin sodium alginate ALG500 and low-endotoxin sodium alginate AL500 was individually implanted in an amount of 1 ml into six rats in a manner similar to that in Example 1. After 24 weeks, HE staining and SOFG staining were performed in the same manner as in Example 1. Fig. 5 shows the results.

The results in Fig. 5 show that in the implantation of ALG500 and the implantation of AL500, the retention was observed. Inflammatory changes were reduced, but they strongly persisted in some cases. No particular differences were found between ALG500 and AL500.

### Example 6

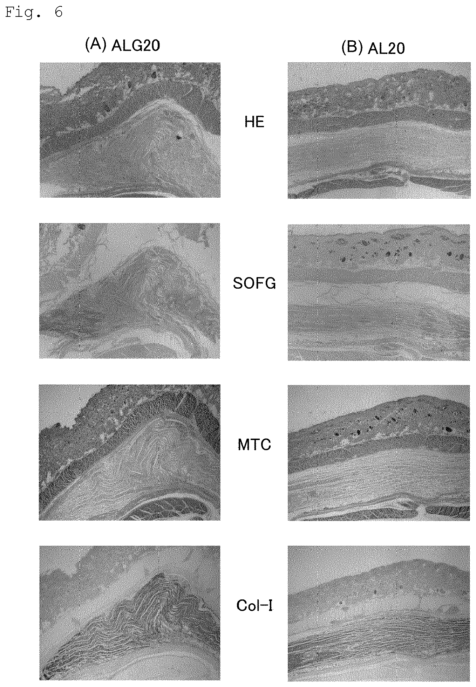

Rats in which implantation was performed in the same manner as in Example 4 were subjected to MTC staining, and type I collagen staining in addition to HE staining and SOFG staining. Fig. 6 shows the results. MTC staining and type I collagen staining were performed according to usual methods.

The results in Fig. 6 show that in both of the implantation of ALG20 and the implantation of AL20, SOFG staining, MTC staining, and type I collagen staining were positive in the sites in which a change in orientation was observed. It was thus believed that collagen tissue etc. were formed and that there was a significant orientation according to collagen tissue tension.

### Example 7

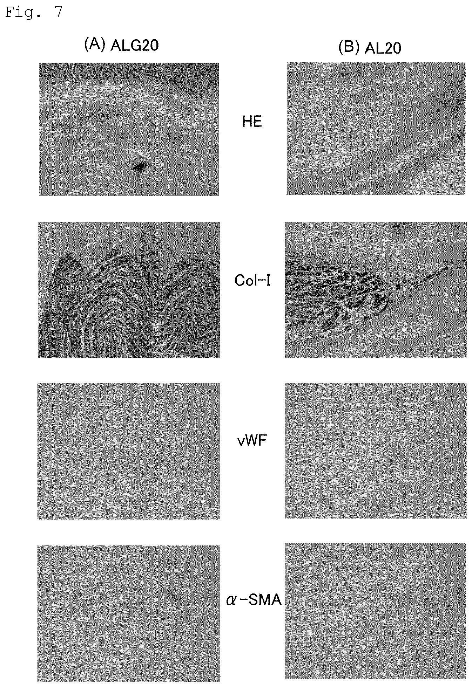

Rats in which implantation was performed in the same manner as in Example 4 were subjected to Col-I staining, vWF staining, and alfa-SMA staining in addition to HE staining. Fig. 7 shows the results. vWF staining and alfa-SMA staining were performed according to usual methods.

The results in Fig. 7 show that in both of the implantation of ALG20 and the implantation of AL20, Col-I staining, vWF staining, and alfa-SMA staining were positive in the sites in which a change in orientation was observed. It was thus believed that subcutaneous adipose tissue and vascular tissue were formed. No scarring changes were observed.

### Example 8

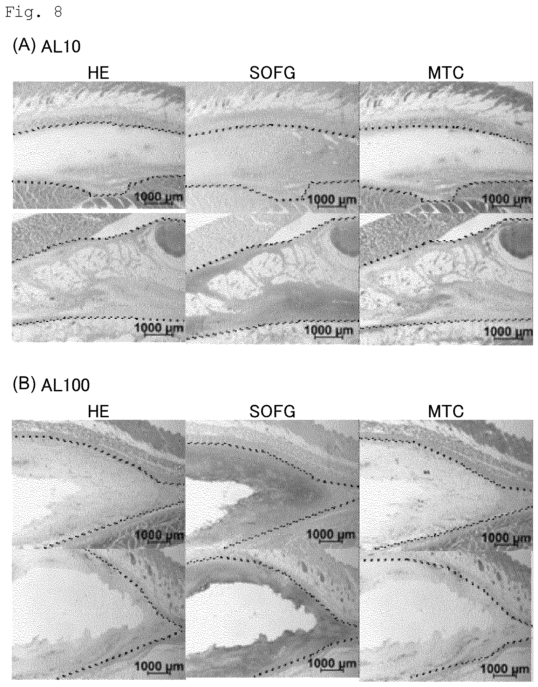

Each of 2% aqueous solutions of low-endotoxin sodium alginate AL10 and low-endotoxin sodium alginate AL100 was individually implanted in an amount of 1 ml into six rats in a manner similar to that in Example 1. After 1 week, HE staining and SOFG staining were performed in the same manner as in Example 1, and further, MTC staining was performed. Fig. 8 shows the results.

The results in Fig. 8 show that in both the implantation of AL10 and the implantation of AL100, residual injected sodium alginate was observed.

### Example 9

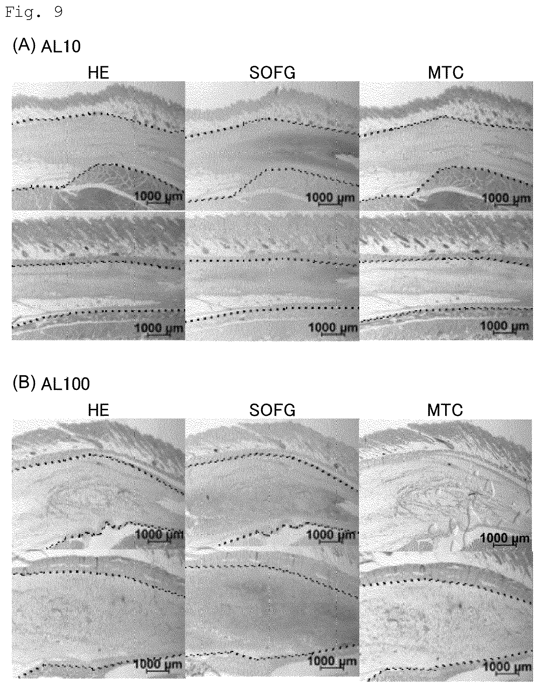

For six rats in which implantation was performed in the same manner as in Example 8, HE staining, SOFG staining, and MTC staining were performed in the same manner as in Example 8, 3 weeks after the implantation. Fig. 9 shows the results.

The results in Fig. 9 show that in both the implantation of AL10 and the implantation of AL100, residual injected sodium alginate was observed.

### Example 10

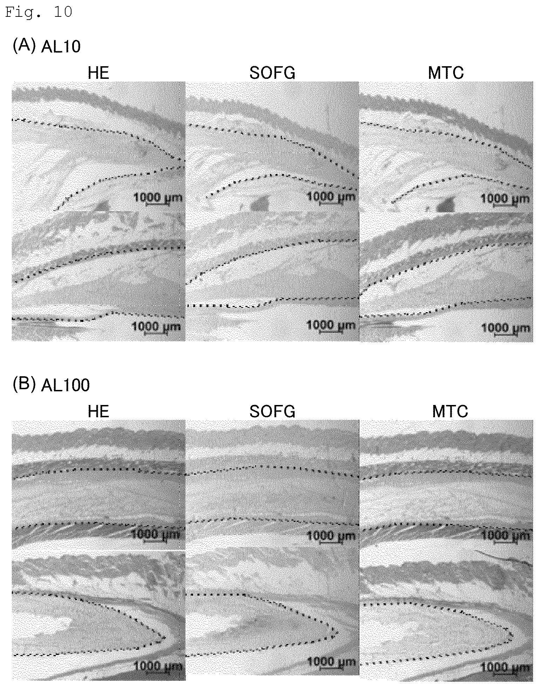

For six rats in which implantation was performed in the same manner as in Example 8, HE staining, SOFG staining, and MTC staining were performed in the same manner as in Example 8, 6 weeks after the implantation. Fig. 10 shows the results.

The results in Fig. 10 show that in the implantation of AL100, residual injected sodium alginate was observed, whereas in the implantation of AL10, the amount of residual sodium alginate was considerably reduced. In addition, in the implantation of AL10, a change in orientation was partially observed.

### Example 11

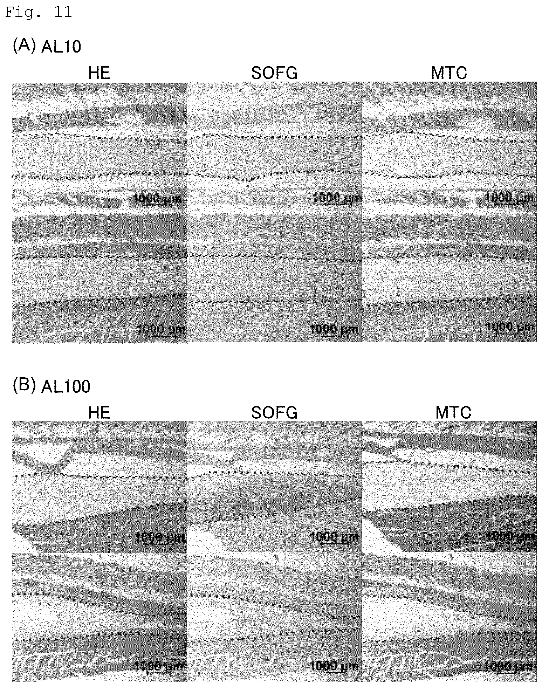

For six rats in which implantation was performed in the same manner as in Example 8, HE staining, SOFG staining, and MTC staining were performed in the same manner as in Example 8, 12 weeks after the implantation. Fig. 11 shows the results.

The results in Fig. 11 show that residual injected sodium alginate was observed in the implantation of AL100, but no residual injected sodium alginate was observed in the implantation of AL10. In addition, in the implantation of AL100, a change in orientation was partially observed, and in the implantation of AL10, a change in orientation was observed.

### Example 12

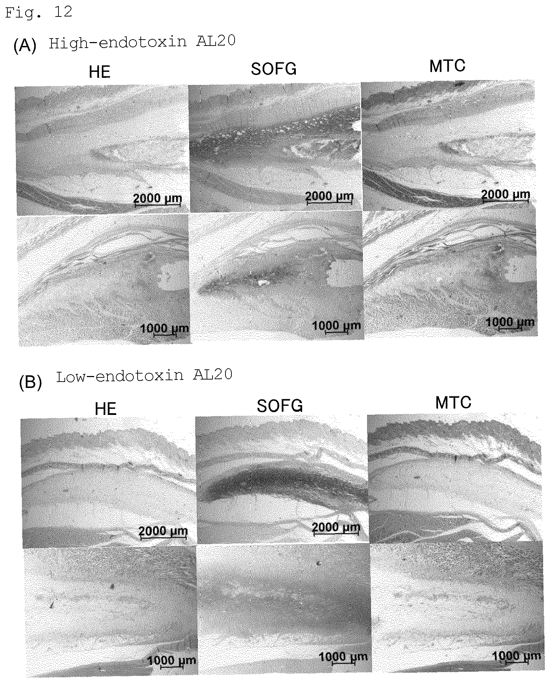

Each of 2% aqueous solutions of high-endotoxin sodium alginate AL20 (high-endotoxin AL20) and low-endotoxin sodium alginate AL20 (low-endotoxin AL20) was individually implanted in an amount of 1 ml into six rats in a manner similar to that in Example 1. After 1 week, HE staining and SOFG staining were performed in the same manner as in Example 1, and MTC staining was performed. Fig. 12 shows the results.

The results in Fig. 12 show that in the implantation of high-endotoxin AL20, inflammatory cell infiltration was observed not only around the implantation sites, but also inside them, whereas in the implantation of low-endotoxin AL20, inflammatory cells were observed only around the implantation sites.

### Example 13

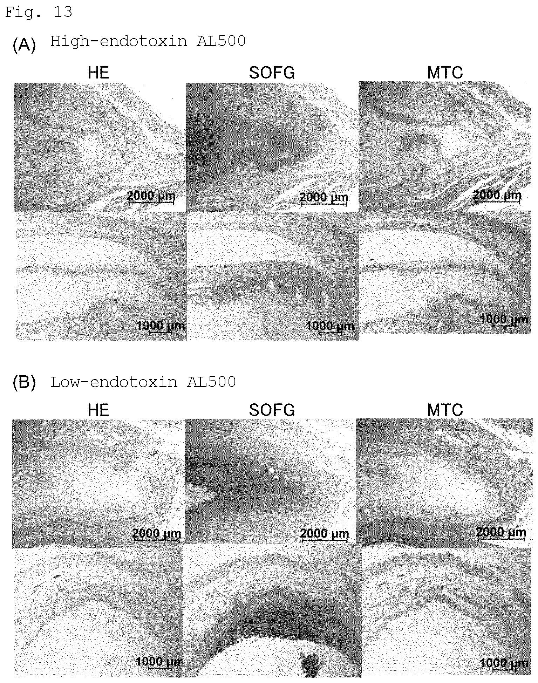

Each of 2% aqueous solutions of high-endotoxin sodium alginate AL500 (high-endotoxin AL500) and low-endotoxin sodium alginate AL500 (low-endotoxin AL500) was individually implanted in an amount of 1 ml into six rats in a manner similar to that in Example 1. After 1 week, HE staining and SOFG staining were performed in the same manner as in Example 1, and MTC staining was performed. Fig. 13 shows the results.

The results in Fig. 13 show that in the implantation of high-endotoxin AL500, inflammatory cell infiltration was observed not only around the implantation sites, but also inside them, whereas in the implantation of low-endotoxin AL500, inflammatory cells were observed only around the implantation sites.

### Example 14

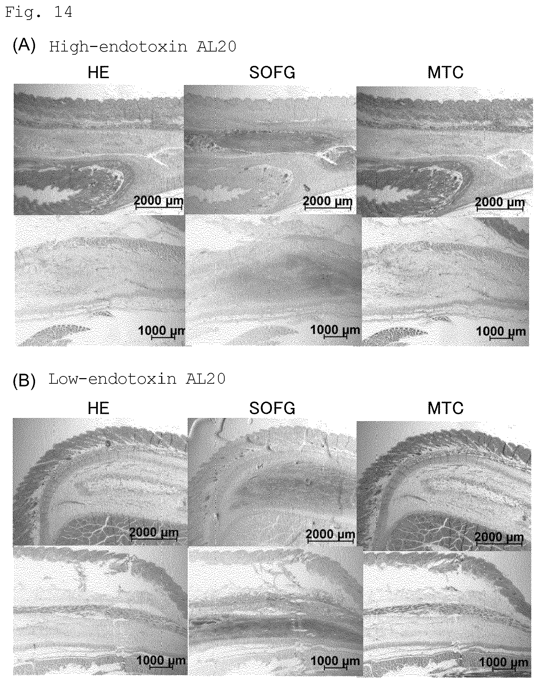

Each of high-endotoxin AL20 and low-endotoxin AL20 was individually implanted into rats in the same manner as in Example 12. After 3 weeks, the same staining as in Example 12 was performed. Fig. 14 shows the results.

The results in Fig. 14 show that more inflammatory cells were observed in the implantation of high-endotoxin AL20 than in the implantation of low-endotoxin AL20.

### Example 15

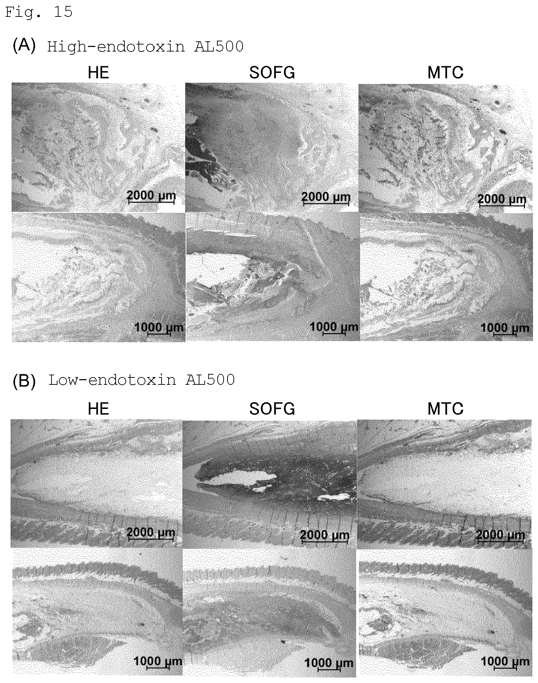

Each of high-endotoxin AL500 and low-endotoxin AL500 was individually implanted into rats in the same manner as in Example 13. After 3 weeks, the same staining as in Example 13 was performed. Fig. 15 shows the results.

The results in Fig. 15 shows that in the implantation of high-endotoxin AL500, inflammatory cell infiltration was observed not only around the implantation sites, but also inside them, whereas in the implantation of low-endotoxin AL500, inflammatory cells were observed only around the implantation sites, as in the results 1 week after the implantation shown in Fig. 13.

### Example 16

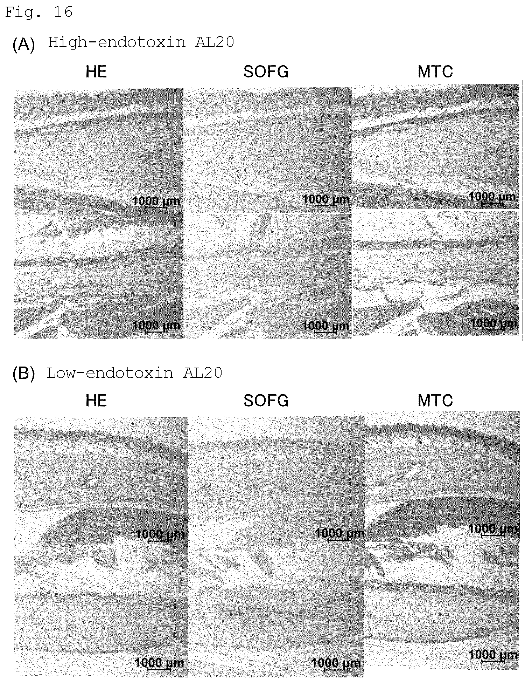

Each of high-endotoxin AL20 and low-endotoxin AL20 was individually implanted into rats in the same manner as in Example 12. After 6 weeks, the same staining as in Example 12 was performed. Fig. 16 shows the results.

The results in Fig. 16 show that in the implantation of high-endotoxin AL20, inflammatory cell infiltration was reduced compared to that 3 weeks after the implantation shown in Fig. 14, and was slightly stronger than in the implantation of low-endotoxin AL20.

### Example 17

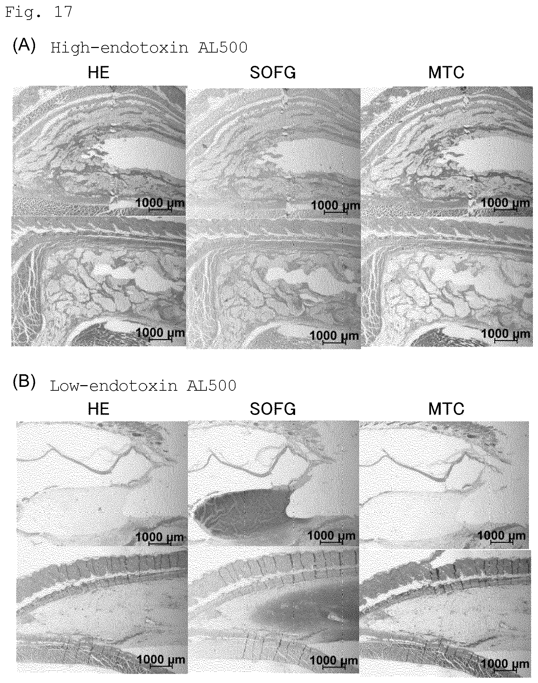

Each of high-endotoxin AL500 and low-endotoxin AL500 was individually implanted into rats in the same manner as in Example 13. After 6 weeks, the same staining as in Example 13 was performed. Fig. 17 shows the results.

The results in Fig. 17 show that in the implantation of high-endotoxin AL500, inflammatory cell infiltration was observed not only around the implantation sites, but also inside them, as in the results 3 weeks after the implantation shown in Fig. 15, whereas in the implantation of low-endotoxin AL500, inflammatory cells were observed only around the implantation sites, as in the results 1 week and 3 weeks after the implantation shown in Figs. 11 and 13.

### Example 18

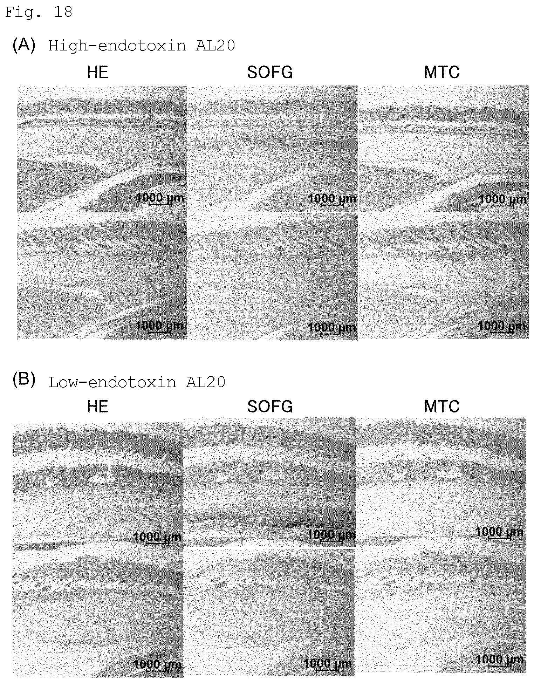

Each of high-endotoxin AL20 and low-endotoxin AL20 was individually implanted into rats in the same manner as in Example 12. After 12 weeks, the same staining as in Example 12 was performed. Fig. 18 shows the results.

The results in Fig. 18 show that in the implantation of high-endotoxin AL20, the same degree of inflammatory cell infiltration as in the implantation of low-endotoxin AL20 was observed. In the implantation of low-endotoxin AL20, inflammatory cells were observed only around the implantation sites, as in the results 1 week and 3 weeks after the implantation shown in Figs. 14 and 16. A significant change in orientation was also confirmed. This significant change in orientation was not observed in the implantation of high-endotoxin AL20.

### Example 19

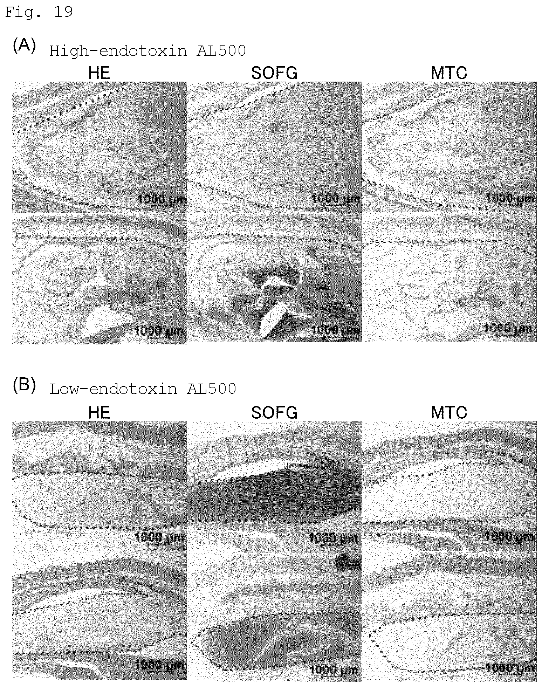

Each of high-endotoxin AL500 and low-endotoxin AL500 was individually implanted into rats in the same manner as in Example 13. After 12 weeks, the same staining as in Example 13 was performed. Fig. 19 shows the results.

The results in Fig. 19 show that in the implantation of high-endotoxin AL500, inflammatory cell infiltration was observed not only around the implantation sites, but also inside them, as in the results 3 weeks and 6 weeks after the implantation shown in Figs 15 and 17. In the implantation of low-endotoxin AL500, inflammatory cells were observed only around the implantation sites, as in the results 1 week and 3 weeks after the implantation shown in Figs. 11 and 13. A significant change in orientation was also confirmed in some sites. This significant change in orientation was not observed in the implantation of high-endotoxin AL500.

### Comparative Example 1

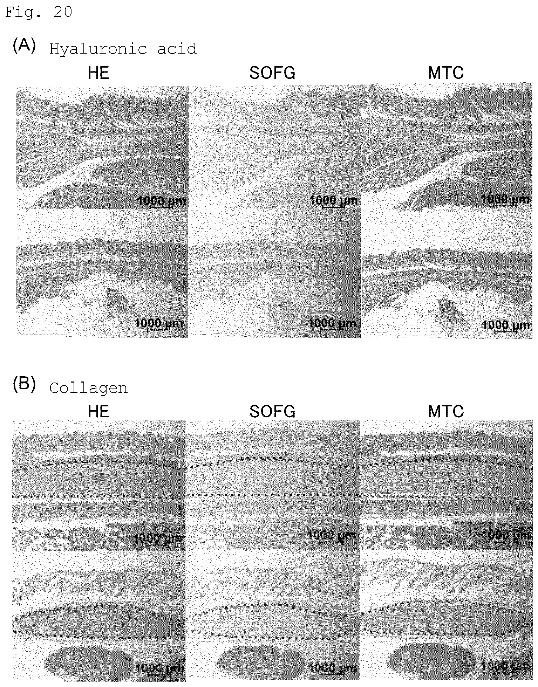

Each of hyaluronic acid (produced by Nichi-Iko Pharmaceutical Co., Ltd.) at a concentration of 25 mg/2.5 ml and collagen at a concentration of 2% was individually implanted in an amount of 1 ml into six rats in a manner similar to that in Example 1. After 1 week, HE staining and SOFG staining were performed in the same manner as in Example 1, and further, MTC staining was performed. Fig. 20 shows the results.

The results in Fig. 20 show that hyaluronic acid disappeared from the injection sites. Collagen was present in an amorphous state at the injection sites, and no cell infiltration was observed.

### Comparative Example 2

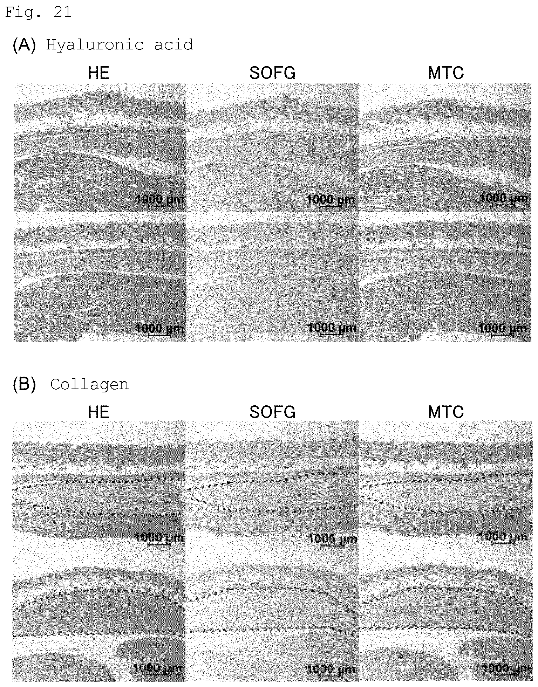

Each of hyaluronic acid and collagen at a concentration of 2% was individually implanted into six rats in the same manner as in Comparative Example 1. After 3 weeks, HE staining, SOFG staining, and MTC staining were performed. Fig. 21 shows the results.

The results in Fig. 21 show that as in the results 1 week after the implantation shown in Fig. 20, hyaluronic acid disappeared from the injection sites; collagen was present in an amorphous state at the injection sites, and no cell infiltration was observed.

### Comparative Example 3

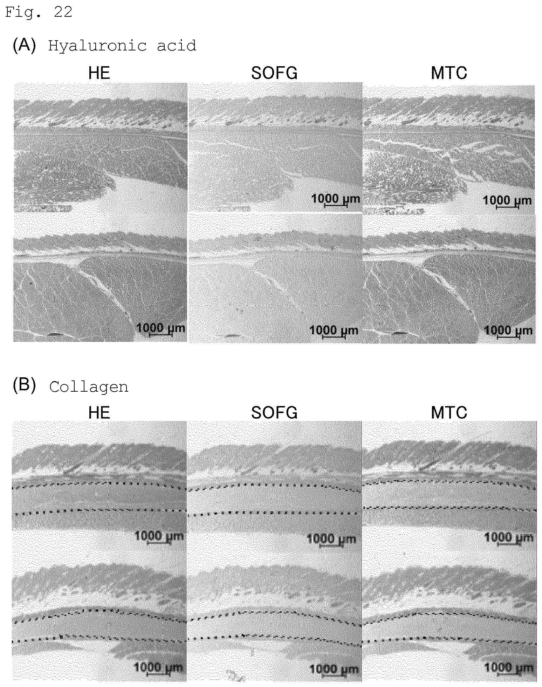

Each of hyaluronic acid and collagen at a concentration of 2% was individually implanted into six rats in the same manner as in Comparative Example 1. After 6 weeks, HE staining, SOFG staining, and MTC staining were performed. Fig. 22 shows the results.

The results in Fig. 22 show that as in the results 1 week and 3 weeks after the implantation shown in Figs. 20 and 21, hyaluronic acid disappeared from the injection sites; collagen was present in an amorphous state at the injection sites, and no cell infiltration was observed.

### Comparative Example 4

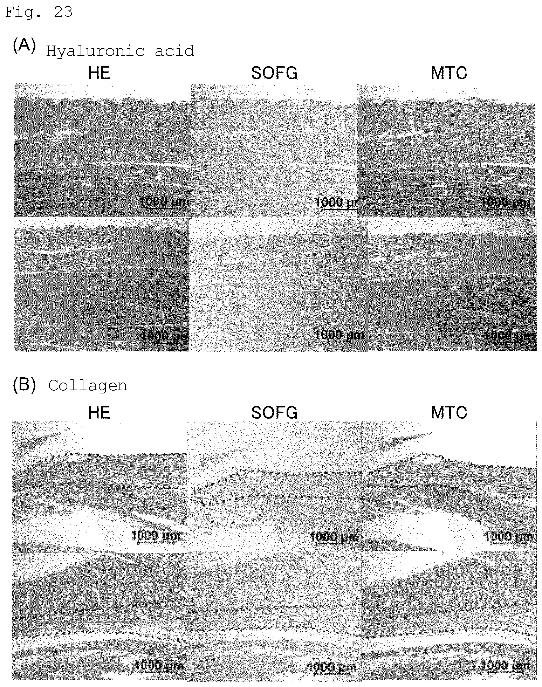

Each of hyaluronic acid and collagen at a concentration of 2% was individually implanted into six rats in the same manner as in Comparative Example 1. After 12 weeks, HE staining, SOFG staining, and MTC staining were performed. Fig. 23 shows the results.

The results in Fig. 23 show that as in the results 1 to 6 weeks after the implantation shown in Figs. 20, 21, and 22, hyaluronic acid disappeared from the injection sites. Collagen was also present in an amorphous state at the injection sites, and no cell infiltration was observed. In the implantation of collagen, although fat cells were observed around the injection sites, no change in orientation was observed.

### Example 20

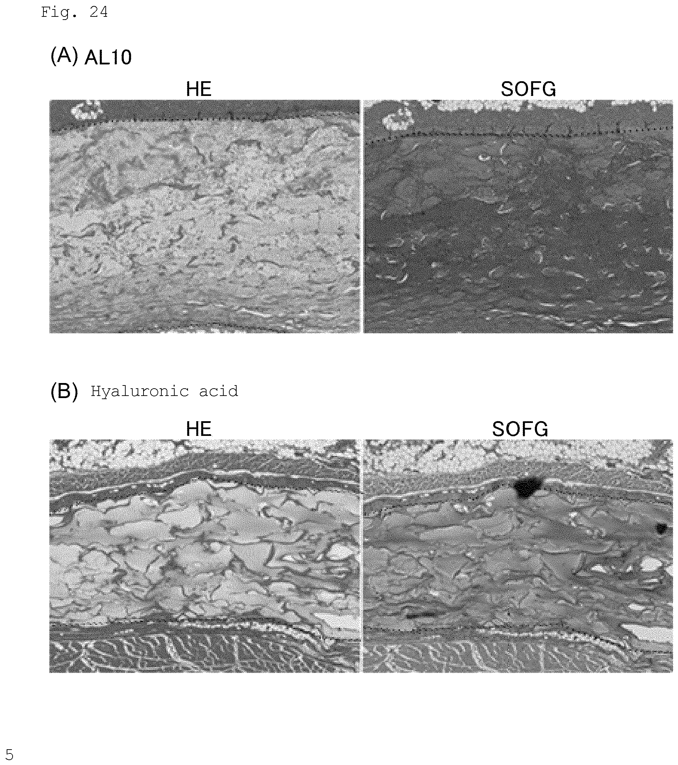

Each of AL10 crosslinked with calcium and TEOSYAL RHA 1 (hyaluronic acid, produced by Teoxane) was individually implanted in an amount of 1 ml into six rats in a manner similar to that in Example 1. After 12 weeks, HE staining and SOFG staining were performed in the same manner as in Example 1. Fig. 24 shows the results.

A total of 1 ml of calcium-crosslinked AL10 was injected using a double syringe in which 0.5 ml of a 4% AL10 solution and 0.5 ml of a calcicol solution diluted to 1/8 with water for injection were simultaneously formed into a single gel at the spout. The results are as described below. In this case, the crosslinking state by calcium is mild.

As shown in Fig. 24, in the implantation of mildly calcium-crosslinked AL10, formation of tissue having an orientation and angiogenesis were observed. On the other hand, in the implantation of hyaluronic acid, hyaluronic acid remained throughout the entire layer, and cell infiltration, formation of tissue having an orientation, or angiogenesis was not observed.

### Example 21

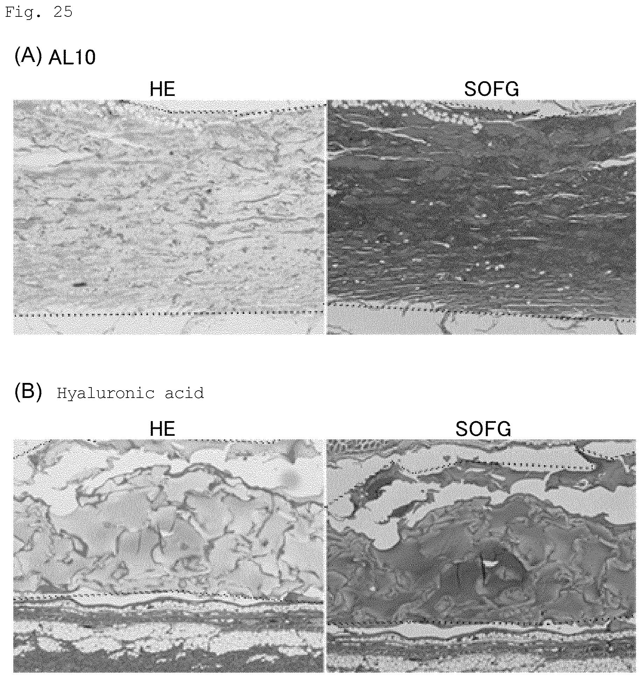

Each of AL10 crosslinked with calcium and TEOSYAL RHA 1 (hyaluronic acid, produced by Teoxane) was individually implanted into six rats in the same manner as in Example 20. After 24 weeks, HE staining and SOFG staining were performed. Fig. 25 shows the results.

As shown in Fig. 25, in the implantation of mildly calcium-crosslinked AL10, formation of tissue having an orientation and angiogenesis were more clearly observed than the results 12 weeks after the implantation shown in Fig. 20. On the other hand, in the implantation of hyaluronic acid, even after 24 weeks, hyaluronic acid remained throughout the entire layer, and cell infiltration, formation of tissue having an orientation, or angiogenesis was not observed, as in the results after 12 weeks shown in Fig. 20.

### Example 22

The following study was conducted on sulfated alginic acid, in which sulfate groups were introduced into alginic acid itself, or a salt thereof, to investigate the tissue-forming effects of using a sulfate group component in combination with alginic acid.

After sulfated sodium alginate at a concentration of 0.5% was subcutaneously injected and implanted in the rat back, histopathological evaluation and study were conducted in the same manner as in Example 1. The sulfated alginic acid used was synthesized by Tokyo Chemical Industry Co., Ltd. and has 0.2 sulfate groups per monosaccharide structure part in sodium alginate having a viscosity of 100 mP/s (concentration: 10 g/L, 20°C) .

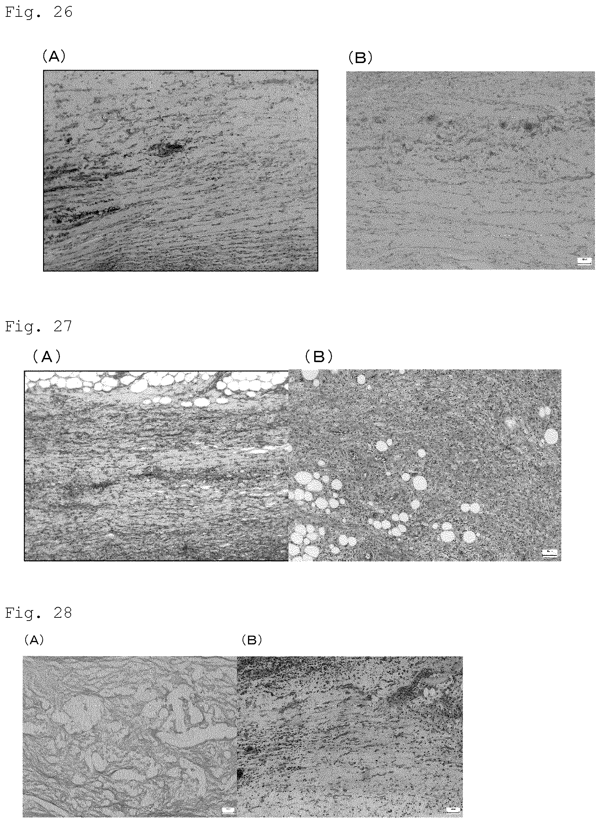

Fig. 26 shows the results of HE staining of the implantation site 1 week after subcutaneous implantation of sulfated sodium alginate. Fig. 26 also shows the results of HE staining of the implantation site after 1 week when conducting the same study using the same sodium alginate into which no sulfate groups were introduced, as a comparison.

The results in Fig. 26 show that in the implantation of sulfated alginic acid, a tissue composed of a significant layered structure of collagen was formed, and a nutrient vascular network and interstitial tissue were also already formed along this collagen layered structure (Fig. 26A). On the other hand, in the implantation of alginic acid into which no sulfate groups were introduced, as the comparison, the layered structure of collagen and the interstitial tissue with a nutrient vascular network were unclear (Fig. 26B). It was thus revealed that tissue formation is further promoted when sulfated alginic acid is used than when ordinary sodium alginate is injected and implanted.

### Example 23

The following study was conducted to investigate the tissue-forming effect of using alginic acid and a sulfate group component in combination.

In a manner similar to that in Example 22, a mixture of AL10 at a concentration of 2% as sodium alginate and sulfated dextran (produced by Sigma-Aldrich) at a concentration of 0.01 mg/mL was subcutaneously implanted in a rat, and after 2 weeks, HE staining of the implantation site was performed. Fig. 27 shows the results. Fig. 27 also shows the results of HE staining of the implantation site after 2 week when conducting the same study using the same sodium alginate alone, which is not mixed with sulfated dextran, as a comparison.

The results in Fig. 27 show that when sulfated alginic acid was mixed, a tissue composed of a layered structure of collagen was already formed, and in addition to a nutrient vascular network and interstitial tissue, subcutaneous adipose tissue was also already formed along this collagen layered structure (Fig. 27A). On the other hand, when alginic acid that is not mixed with sulfated dextran was used as the comparison, the layered structure of collagen and the interstitial tissue with a nutrient vascular network were unclear (Fig. 27B). It was thus revealed that tissue formation is also further promoted when sodium alginate mixed with sulfated dextran is used than when ordinary sodium alginate is injected and implanted.

### Example 24

The following study was conducted to confirm the tissue-filling effect of alginic acid and the like.

As highly calcium-crosslinked alginic acid, a sample obtained by mixing sodium alginate (AL10) at a concentration of 1% with the same volume of a calcicol stock solution was injected and implanted into rat subcutaneous tissue in a manner similar to that of Example 22, and after 1 week, HE staining of the implantation site was performed. Fig. 28 shows the results. Fig. 28 also shows the results of HE staining of the implantation site after 1 week when conducting the same study using the same concentration (i.e., concentration: 0.5%) of the same sodium alginate alone as a comparison.

The results in Fig. 28 show that when highly calcium-crosslinked alginic acid was injected, although crosslinked alginic acid was present in an aggregated form at the injection site, there was little infiltration of cells and nutrient vessels inside it (Fig. 28A), as seen in collagen of a Comparative Example (Fig. 23B) and hyaluronic acid of a Comparative Example (Fig. 24B), both of which are conventional tissue fillers. That is, it was revealed that highly crosslinked alginic acid does not act as a tissue-forming agent, but acts as a tissue filler. On the other hand, when the same concentration of the same sodium alginate was implanted as the comparison, significant cell infiltration was observed in a site in which alginic acid was present, and initiation of formation of a layered structure of collagen and interstitial tissue with a nutrient vascular network was observed (Fig. 28B).

### Example 25

The following study was conducted to confirm the tissue scaffolding effect of alginic acid.

A PGA non-woven fabric (Neoveil (trademark) Sheet, Gunze Medical Limited) was used as a tissue reinforcement material, and a gel of sodium alginate (AL10) mildly crosslinked with calcium (i.e., a gel obtained by mixing equal volumes of a 4% AL10 solution and a calcicol solution diluted to 1/8 with water for injection), which is the same as that described in Example 20, was used as alginic acid.

The PGA non-woven fabric and the gel were combined and implanted in rat peritoneal submesothelial tissue. In addition, a 1% poly-L-lysine solution was used as a crosslinking agent in place of calcium (the calcicol solution); specifically, a gel was obtained by mixing equal volumes of a 4% AL10 solution and a solution of 1% poly-L-lysine dissolved in distilled water for injection, and the gel and a PGA non-woven fabric were combined and implanted in rat peritoneal submesothelial tissue. As controls, only a gel obtained by mixing equal volumes of a 4% AL10 solution and a calcicol solution diluted to 1/8 with water for injection was implanted in peritoneal submesothelial tissue without combining with a non-woven fabric, and only a non-woven fabric was implanted. Fig. 29 show the results of HE staining of the implantation sites 4 weeks after the implantation of these.

As shown in Figs. 29A and 29B, when alginic acid was used in combination, peritoneal submesothelial tissue was generated with a sufficient thickness, residual alginic acid was observed around the fibers of the non-woven fabric, and a rich nutrient vascular network and increased infiltration of interstitial cells were observed in the residual alginic acid. On the other hand, when the non-woven fabric alone was implanted without using alginic acid, the thickness of the formed peritoneal submesothelial tissue was very thin, and slight tissue formation was only observed around the fibers of the non-woven fabric of PGA and the like (Fig. 29C). When only calcium-crosslinked alginic acid was implanted without using a non-woven fabric, only regeneration of thin peritoneal tissue was observed (Fig. 29D).

The mechanical strength of a gel scaffold made of calcium-crosslinked alginic acid alone and a combination of a PGA non-woven fabric or LA/CL non-woven fabric with the above gel as scaffold materials was compared. The gel scaffold made of calcium-crosslinked alginic acid alone as a sample for measuring the mechanical strength of a scaffold was prepared by injecting, into a silicone container with a width of 10 mm and a length of 30 mm, a gel (total amount: 1 ml) by simultaneously ejecting 0.5 ml of a 4% AL10 solution and 0.5 ml of a calcicol solution diluted to 1/8 with water for injection, using a double syringe in which these solutions were formed into a single gel at the spout. The scaffold of the combination of a PGA non-woven fabric or LA/CL non-woven fabric with the above gel as a sample for measuring the mechanical strength of a scaffold was prepared by placing a double layer of PGA non-woven fabric or LA/CL non-woven fabric beforehand on the bottom of a silicone container and simultaneously ejecting 0.5 ml of a 4% AL10 solution and 0.5 ml of a calcicol solution diluted to 1/8 with water for injection on the non-woven fabric. The 10-mm-width portions on both sides of each sample were held with jigs, the sample was pulled to both sides, and the breaking strength was measured using the following apparatuses.
- Load measuring instrument: desktop load measuring instrument MODEL-1356R (Aikoh Engineering Co., Ltd.)
- Force gauge: MODEL-RX-10 (Aikoh Engineering Co., Ltd.)
- Hardware: PowerLab 2/26 (ADInstruments)
- Software: LabChart (ADInstruments)

Both of the 10-mm-width scaffolds that were prepared by combining a PGA non-woven fabric or LA/CL non-woven fabric with the crosslinked alginic acid gel had a breaking strength of 2 N or more. On the other hand, the breaking strength of the crosslinked alginic acid gel alone was 0.1 N or less. It was thus revealed that when a low-endotoxin alginic acid component is combined with a conventional tissue reinforcement material, an excellent scaffold material that has an excellent tensile strength and a function of actively forming a tissue having a sufficient thickness is obtained.

### Example 26

2-year-old male or female beagle dogs weighing approximately 10 kg were subjected to laparotomy under general anesthesia, and the stomach was incised to expose the mucosal surface. A total of 4 ml of each of alginic acid preparations (including those containing sodium polyglutamate) was individually injected into the submucosal layer. After the incised stomach was sutured closed, the open abdominal wound was sutured closed. After 4 weeks, the stomach was removed from each beagle dog, the stomach wall at the alginic acid injection site was formalin-fixed, and then histopathological microscopic specimens were prepared using a usual method and subjected to HE staining for evaluation. A control experiment in which no injection treatment of an alginic acid preparation was performed was also conducted. Fig. 30 shows the results.

Fig. 30A is an HE stained image of the control. Fig. 30B is an HE stained image of a site in which relatively low-molecular-weight sodium alginate (molecular weight: 10000 or less) was injected, and some tissue regeneration was observed in the submucosal layer compared with the control shown in Fig. 30A. Fig. 30C is an HE stained image of a site in which AL10 at a concentration of 0.5% was injected, and significant tissue regeneration was observed in the submucosal layer. Fig. 30D is an HE stained image of a site in which 2 ml of relatively low-molecular-weight alginic acid (molecular weight: 10000 or less) and 2 ml of a 0.5% sodium polyglutamate solution were injected in the same site, and more significant regeneration of the submucosal layer was observed than in the case of sodium alginate shown in Fig. 30B. Fig. 30E is an HE stained image of a site in which 2 ml of 0.5% AL10 and 2 ml of a 0.5% sodium polyglutamate solution were injected in the same site, and more significant tissue regeneration of the submucosal layer was observed than in the case of AL10 alone shown in Fig. 30C.

## Claims

1. A tissue-forming agent comprising alginic acid or a salt thereof.

2. The forming agent according to claim 1, wherein the tissue is an epithelial tissue, a tissue lining an epithelial tissue layer, or a tissue located deeper than these tissues.

3. The forming agent according to claim 1 or 2, wherein the amount of endotoxin contained is less than or equal to 1500 EU/mL or /mg.

4. The forming agent according to claim 1 or 2, wherein the alginic acid or a salt thereof has a viscosity of 0.1 to 1500 mPa/s.

5. The forming agent according to claim 1 or 2, wherein the alginic acid or a salt thereof has an M/G ratio of 0.001 to 0.999.

6. The forming agent according to claim 1 or 2, wherein the alginic acid or a salt thereof is crosslinked with calcium or polylysine.

7. The forming agent according to claim 1 or 2, wherein the alginic acid or a salt thereof is sulfated.

8. The forming agent according to claim 1 or 2, further comprising a sulfated substance.

9. A tissue filler comprising alginic acid or a salt thereof.

10. A tissue scaffolding agent comprising alginic acid or a salt thereof.
